(19) Europäisches Patentamt
European Patent Office
Office européen des brevets

(11) EP 4 102 292 A1

(12) EUROPEAN PATENT APPLICATION
published in accordance with Art. 153(4) EPC

(43) Date of publication:
**14.12.2022 Bulletin 2022/50**

(21) Application number: **20917679.1**

(22) Date of filing: **27.10.2020**

(51) International Patent Classification (IPC):
***G02C 7/04*** (2006.01) ***A61L 12/08*** (2006.01)
***C08K 5/12*** (2006.01) ***C08K 5/5415*** (2006.01)
***C08K 5/092*** (2006.01) ***C08L 33/14*** (2006.01)
***C08J 7/04*** (2020.01) ***C09D 133/14*** (2006.01)

(52) Cooperative Patent Classification (CPC):
**A61L 12/08; C08J 7/04; C08K 5/092; C08K 5/12; C08K 5/5415; C08L 33/14; C09D 133/14; G02C 7/04**

(86) International application number:
**PCT/KR2020/014709**

(87) International publication number:
**WO 2021/157814 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.02.2020 KR 20200012593**
**20.10.2020 KR 20200136049**

(71) Applicant: **Yoon, Kyung Tae**
**Gimpo-si, Gyeonggi-do 10073 (KR)**

(72) Inventor: **Yoon, Kyung Tae**
**Gimpo-si, Gyeonggi-do 10073 (KR)**

(74) Representative: **Cabinet Chaillot**
**16/20, avenue de l'Agent Sarre**
**B.P. 74**
**92703 Colombes Cedex (FR)**

# (54) CONTACT LENS AND MANUFACTURING METHOD THEREFOR

(57) The present invention relates to a contact lens and a manufacturing method thereof. The contact lens according the present invention may include a resin composition containing an acrylic-based monomer and a Boswellia extract. By containing a Boswellia extract, the contact lens according to an embodiment of the present invention can prevent adherence of bacteria when worn on the user's eye and serve as an antibacterial agent to prevent bacterial infection, so it can secure a good contact fit and high antibacterial performance.

EP 4 102 292 A1

## Description

### TECHNICAL FIELD

The present invention relates to a contact lens and a manufacturing method thereof, and particularly to a contact lens with a good contact fit and high stability, and a manufacturing method thereof.

### BACKGROUND ART

Contact lenses are thin lenses that can be directly attached to the corneal surface of the eye, and are broadly divided into hard and soft contact lenses. Recently, silicone hydrogel lenses that combine the advantages of a silicone material (hard lens) with high oxygen permeability and a hydrogel material (soft lens) with high flexibility have been produced the most.

Contact lenses may also be classified into vision correction, therapeutic, and cosmetic contact lenses depending on their functional benefits for patients.

As contact lenses come in direct contact with the eyeball, they need to maintain transparency and surface wettability, secure an adequate supply of oxygen from the atmosphere and allow carbon dioxide smoothly released from the cornea. In addition, contact lenses should be designed in consideration of clinical aspects such as smoothing flow of tears and avoiding excessive frictions with the eyelids and the eye surface.

That is, contact lenses are to meet the requirements of tensile strength, biocompatibility, non-toxicity, optical transparency, refractive index, surface wettability, water content for cornea, welling rate, oxygen permeability, and so forth.

Unlike eyeglasses, contact lenses are worn in direct contact with the eyeballs. This requires the users to maintain contact lens hygiene and compliance. In fact, wearing contact lenses for a long time causes the components of tears to adhere to the surface of the contacts, making the contacts uncomfortable to wear, impairing vision, discoloring the contacts, and resulting in the risk of bacterial infection. Besides, protein deposition and external contamination by environmental factors such as fine dust possibly promotes adhesion and proliferation of various microbes on the contacts.

The eye is made up of numerous nerve cells and various types of fibrous materials and more highly susceptible to exposure to bacteria than any other body tissues. In addition, the eye has a high humidity and adequate temperature environment and is thus vulnerable to contamination with airborne dust and microbes. This triggers bacterial infection and eye diseases, resulting in occurrence of rapid and multiple symptoms.

Bacteria commonly associated with eye disorders are fungi, *Pseudomonas aeruginosa,* and *Staphylococcus aureus.* The eye disorders linked to the bacterial infection include fungal corneal ulcers caused by fungi inhabiting dust and clothes, pseudomonas corneal ulcer caused by contaminated lens storage containers, acute catarrhal conjunctivitis and chronic bacterial conjunctivitis caused by *Staphylococcus aureus,* and the like.

For solving these problems, research studies in association with the use of antibacterial substances have been actively conducted.

Korea Patent Registration Publication No. 10-0878053 discloses a contact lens containing naringin as a natural antibacterial agent in order to control bacteria occurring in the eye.

Korea Patent Registration Publication No. 10-1953474 describes a contact lens comprising a polymer matrix containing polymer cavities arranged to recognize a natural antibacterial agent through the molecularly imprinted polymer technique, and glycyrrhizic acid as a natural antibacterial agent.

### SUMMARY OF THE DISCLOSURE

The present invention is to provide a contact lens with a good contact fit and high stability and a manufacturing method thereof.

In accordance with one embodiment of the present invention, there is provided a contact lens that includes a resin composition containing an acrylic-based monomer and a Boswellia extract.

The Boswellia extract may include at least one of boswellic acids represented by the following chemical formulas:

[Chemical Formula 1]

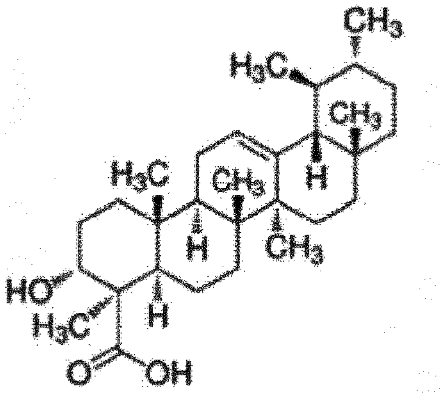

[Chemical Formula 2]

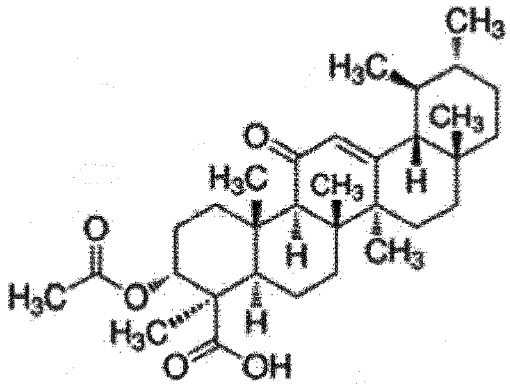

[Chemical Formula 3]

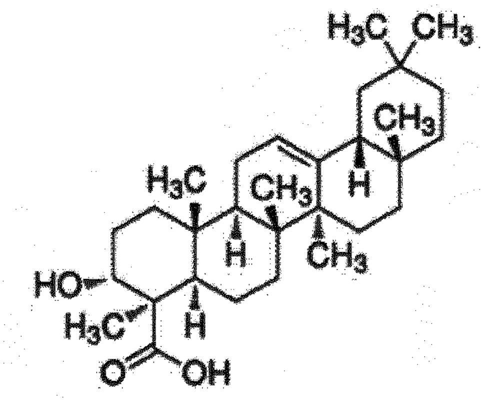

[Chemical Formula 4]

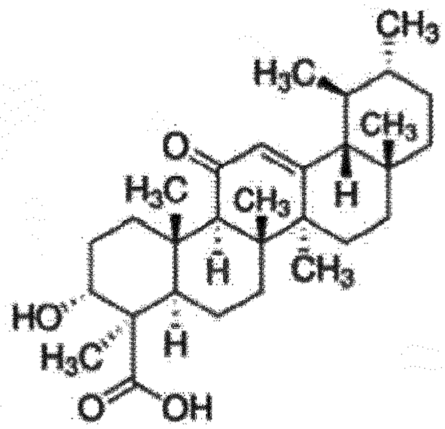

[Chemical Formula 5]

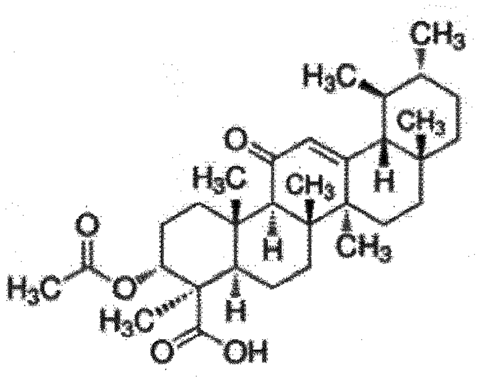

[Chemical Formula 6]

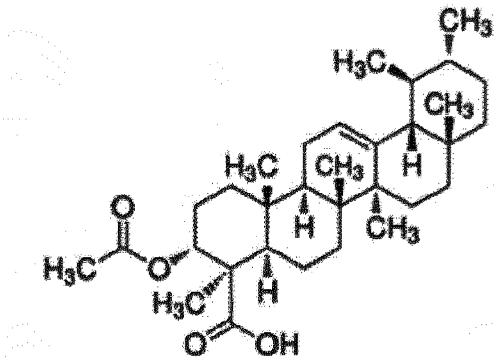

[Chemical Formula 7]

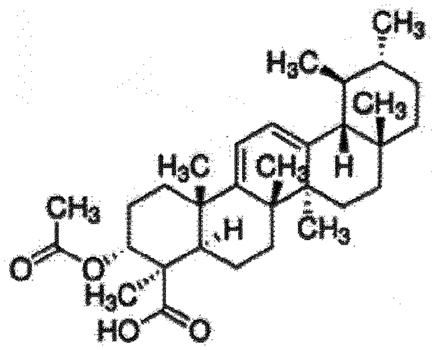

[Chemical Formula 8]

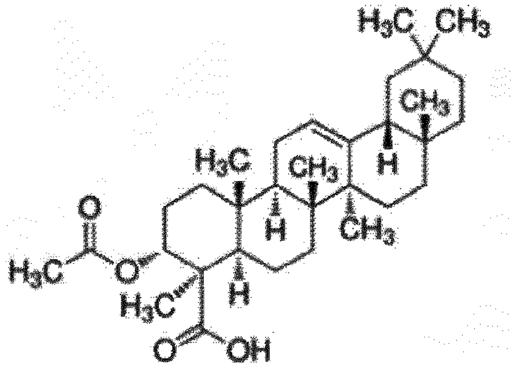

In accordance with an embodiment of the present invention, the acrylic-based monomer may include at least one selected from the group consisting of acrylic acid; methacrylic acid; acrylamide; $C_1$-$C_{15}$ saturated or unsaturated alkyl acrylate or methacrylate; $C_1$-$C_{15}$ hydroxyalkyl acrylate or methacrylate with one to three substituted hydroxyl moieties; and N,N-di($C_1$-$C_{15}$ saturated or unsaturated alkyl)acrylamide.

In accordance with an embodiment of the present invention, the resin composition may further include at least one hydrophilic monomer selected from N-vinylpyrrolidone (NVP) and N-methylpyrrolidone (NMP).

In accordance with an embodiment of the present invention, the resin composition may include a silicone compound.

In accordance with an embodiment of the present invention, the resin composition may include at least one silicone compound selected from the group consisting of 2-(trimethylsilyloxy)ethyl methacrylate, tris(3-methacryloxypropyl)silane, 3-tris(trimethylsiloxy)silylpropyl methacrylate, and 4-methacryloxybutyl terminated polydimethyl siloxane.

In accordance with another embodiment of the present invention, there is provided a method for manufacturing a contact lens that includes: preparing a resin composition containing an acrylic-based monomer; mixing a Boswellia extract in the resin composition; and injecting the mixture into a mold to form a contact lens.

In accordance with still another embodiment of the present invention, there is provided a method for manufacturing a contact lens that includes: preparing a resin composition containing an acrylic-based monomer; injecting the resin composition into a mold to pre-mold a contact lens; and applying a Boswellia extract to the pre-molded contact lens by coating.

In accordance with an embodiment of the present invention, the Boswellia extract may include at least one of boswellic acids represented by the following chemical formulas:

[Chemical Formula 1]

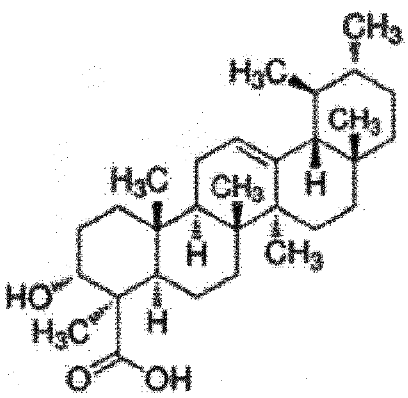

[Chemical Formula 2]

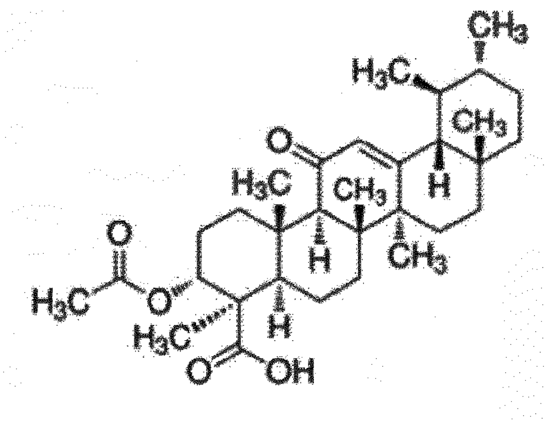

[Chemical Formula 3]

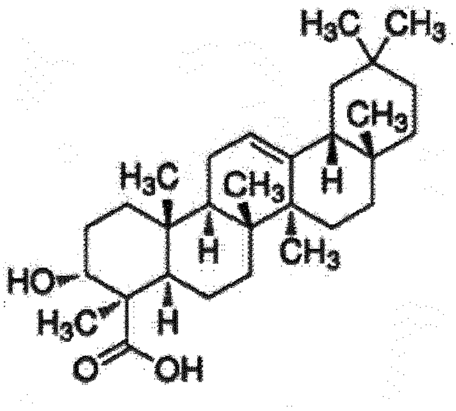

[Chemical Formula 4]

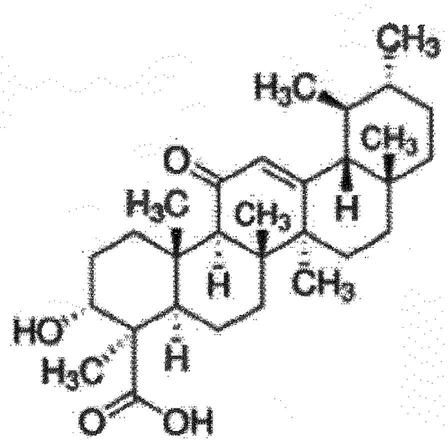

[Chemical Formula 5]

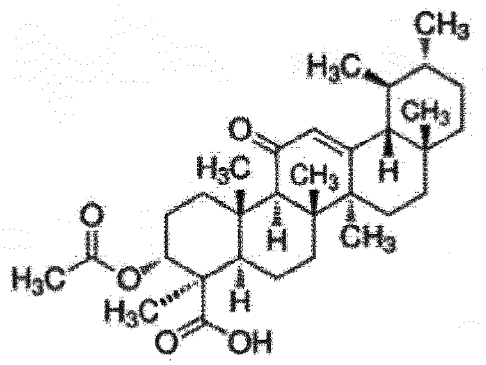

[Chemical Formula 6]

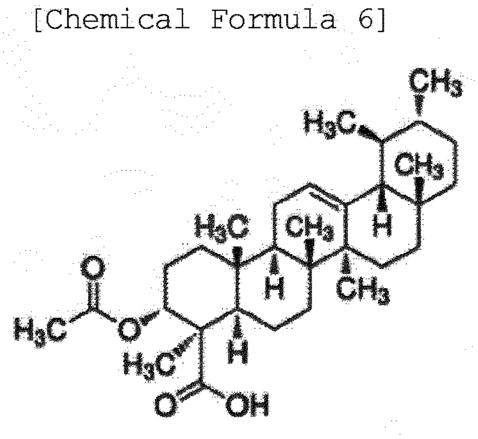

[Chemical Formula 7]

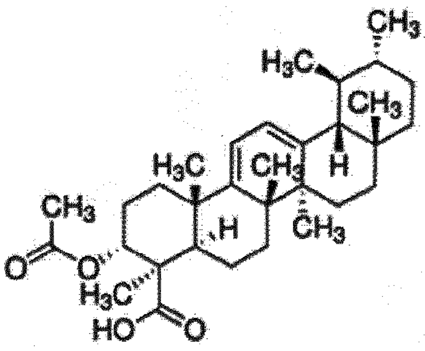

[Chemical Formula 8]

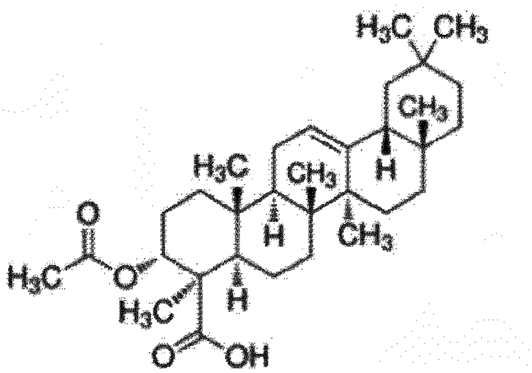

In accordance with an embodiment of the present invention, the step of applying the Boswellia extract by coating may be conducted using a Boswellia coating solution containing 80 to 90 parts by weight of purified water, 0.1 to 2 parts by weight of a hydrophilic polymer, 1 to 5 parts by weight of polyethylene glycol, 0.01 to 5 parts by weight of a Boswellia extract, and 1 to 5 parts by weight of a pH adjusting agent.

In accordance with further another embodiment of the present invention, there is provided a contact lens manufactured by the method.

The contact lens according to an embodiment of the present invention contains a Boswellia extract, so it can secure a good contact fit and high stability.

The contact lens manufactured according to an embodiment of the present invention also contains a Boswellia extract, thereby preventing adherence of bacteria when worn on the user's eye. The Boswellia extract is gradually released from the contact lens and functions as an antibacterial agent to prevent bacterial infection.

The contact lens according to an embodiment of the present invention is coated with the Boswellia extract, which may improve the slip performance of the contact's surface. This makes it possible to reduce a risk of damaging the eyelids and the cornea, provide a protective coating for the surfaces of the eyelids and the cornea to increase the user's eye comfort, and form a stabilized tear film.

Besides, it reduces the protein precipitates adhered to the contact lens to eventually prevent deterioration of the contact's water content and oxygen permeability and results in a decrease in the contact angle, preventing contact lens wear uncomfortable.

## BEST MODES FOR CARRYING OUT THE INVENTION

Hereinafter, a detailed description will be given as to the examples of the present invention in order for those skilled in the art to embody the present invention with ease.

Many modifications and variations are possible, and the embodiments of the present invention disclosed herein are not construed to limit the scope of the invention.

Through the whole document, the term "on" that is used to designate a position of one element with respect to another element includes both a case that the one element is adjacent to the other element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise. The term "about or approximately" or "substantially" are intended to have meanings close to numerical values or ranges specified with an allowable error and intended to prevent accurate or absolute numerical values disclosed for understanding of the present disclosure from being illegally or unfairly used by any unconscionable third party. Through the whole document, the term "step of" does not mean "step for".

The present invention is directed to a contact lens comprising a resin composition containing an acrylic-based monomer and a Boswellia extract, and a manufacturing method thereof.

The term "contact lens" or "contact" as used in the whole document refers to a structure that can be positioned on or in the user's eye. The contact lens may not only be used to correct, improve, or change the user's field of vision, but also be used for other purposes, such as cosmetic purposes. The contact lens may include any material known in the related art and may be a soft, hard, or hybrid lens.

The contact lens according to an embodiment of the present invention contains a Boswellia extract, so it may secure a good contact fit and high stability.

In an embodiment of the present invention, the term "Boswellia extract" refers to the extract of plants belonging to the genus Boswellia. The Boswellia extract may include a boswellic acid as an active ingredient.

Examples of the plants belonging to the genus Boswellia may include, but are not limited to, Boswellia serata, Boswellia frereana, or Boswellia carterii. Specifically, the plants belonging to the genus Boswellia may be Boswellia serata.

Boswellia is rich in ingredients acting on inflammation, such as terten, Boswellic acids, and incensole acetate, which are known to serve as antioxidants and have an excellent effect in treating inflammation. In particular, boswellic acids inhibit the action of an enzyme called 5-lipoxygenase for producing leukotriene from arachidonic acid, so they are known to be far more effective than typical elastase inhibitors such as ursolic acid.

Specifically, there is a case study suggesting that the Boswellia extract reduced the activity of factors triggering inflammation in osteoarthritis (Journal of the Korean Society of Food Science and Nutrition, 43(5), 20145, 631-640 (10 pages)).

Another research study revealed that administration of the Boswellia extract to patients with inflammatory bowel disease (IBD) for 6 weeks resulted in curing 82% of the patients (British Journal of Pharmacy, Francesca Borrelin et al.).

In accordance with an embodiment of the present invention, the method for obtaining the Boswellia extract is not specifically limited.

The method may include, but is not limited to, using an extraction process. For example, the Boswellia extract may be obtained from Boswellia using an extraction solvent, such as water, $C_1$-$C_4$ anhydrous or hydrous lower alcohols (e.g., methanol, ethanol, propanol, or butanol), a mixed solvent of lower alcohol and water, acetone, ethyl acetate, chloroform, 1,3-butylene glycol, or butyl acetate. Specifically, the extraction solvent may be water or ethanol.

In the process of obtaining the Boswellia extract, extraction may be performed with any extractor commonly used in the related art, such as an extractor equipped with a condenser, which is suitable for preventing active ingredients

from evaporating during extraction. In addition, drying of the extract may be performed through a variety of methods known in the related art, such as using a rotatory vacuum evaporator.

In accordance with an embodiment of the present invention, the Boswellia extract may also be subjected to a conventional purification process, such as using an active fraction obtained by a variety of separate purification methods, which may include, but are not limited to, separation using an ultrafiltration membrane with a constant molecular weight cut-off value or various chromatographs (designed for separation according to size, charge, hydrophobicity, or affinity).

In accordance with an embodiment of the present invention, the Boswellia extract may be prepared in a powder form through an additional process, such as vacuum distillation, freeze drying, or spray drying.

The Boswellia extract may include at least one of boswellic acids represented by the following chemical formulas:

[Chemical Formula 1]

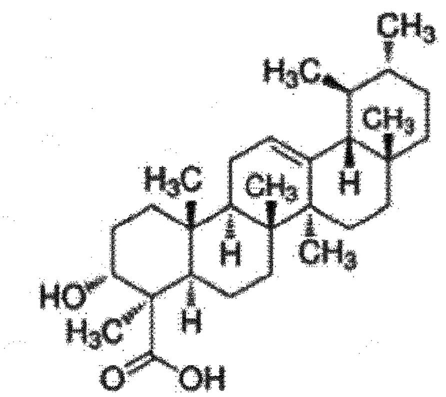

[Chemical Formula 2]

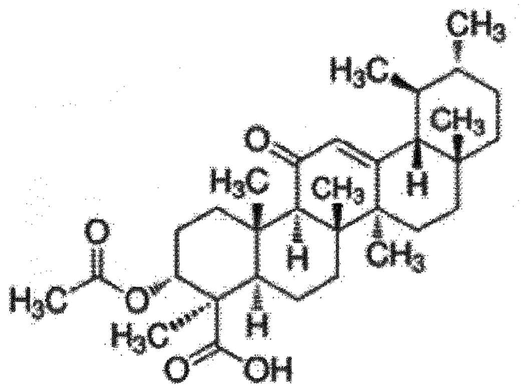

[Chemical Formula 3]

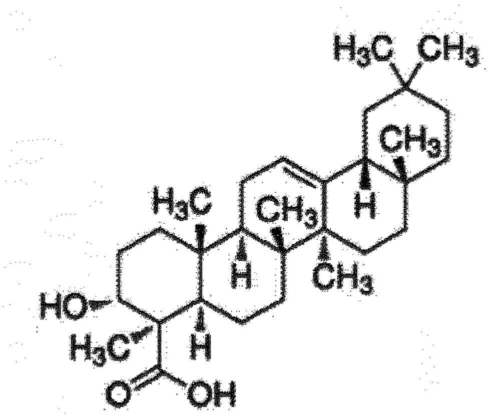

[Chemical Formula 4]

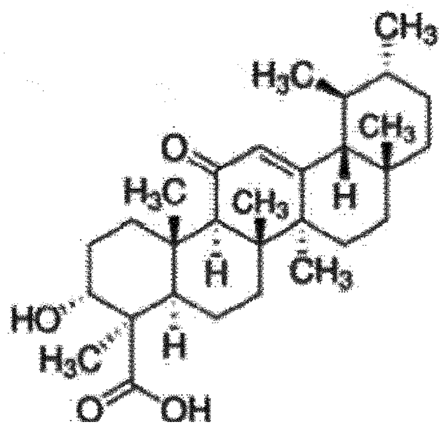

[Chemical Formula 5]

[Chemical Formula 6]

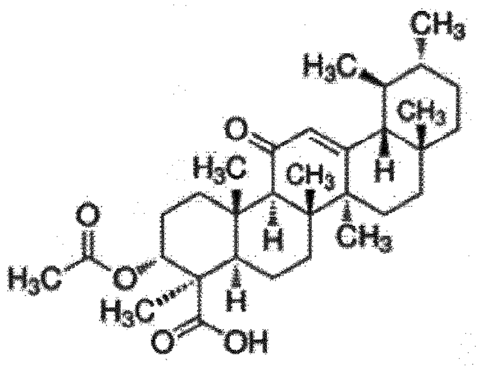

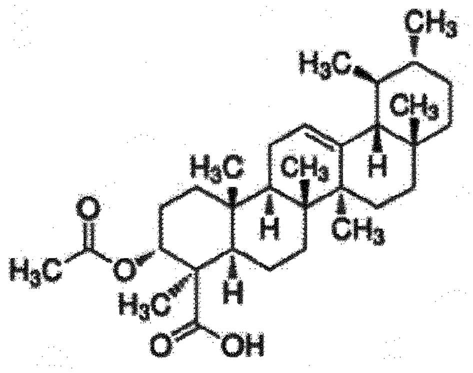

[Chemical Formula 7] [Chemical Formula 8]

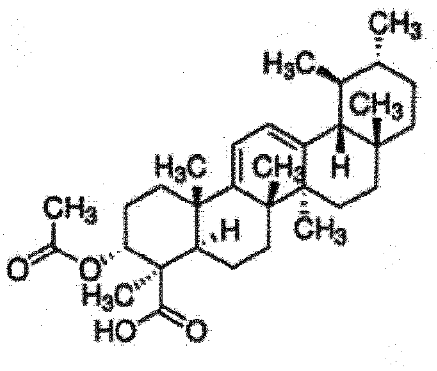

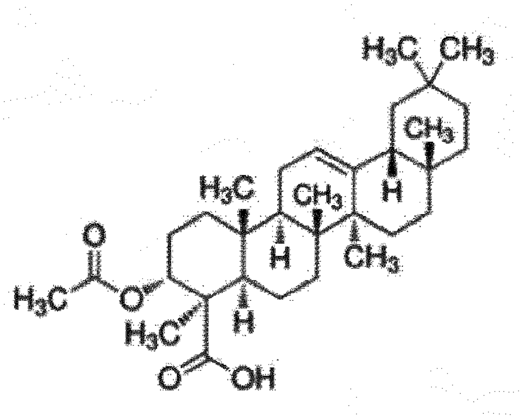

The chemical formula 1 is named β-boswellic acid; the chemical formula 2 is 3-O-acetyl-11-keto-β-boswellic acid; the chemical formula 3 is α-boswellic acid; the chemical formula 4 is 11-keto-β-boswellic acid; the chemical formula 5 is 3-acetyl-11-keto-β-boswellic acid; the chemical formula 6 is 3-O-acetyl-β-boswellic acid; the chemical formula 7 is 3-0-acetyl 9,11-dihydro β-boswellic acid; and the chemical formula 8 is 3-O-acetyl-α-boswellic acid.

The other components constituting the contact lens will be described in the under-mentioned method for manufacturing a contact lens.

Hereinafter, a manufacturing method for contact lens according to one embodiment of the present invention will be described specifically. The under-mentioned method for manufacturing a contact lens may be construed as a method that specifies the contact lens according to an embodiment of the present invention.

In accordance with an embodiment of the present invention, the Boswellia extract may be mixed with the resin composition constituting the contact lens and subjected to polymerization, or applied to the molded contact lens by coating. Hereinafter, each procedure of the manufacturing method will be described in a step-by-step manner.

A method for manufacturing a contact lens according to an embodiment of the present invention may include: preparing a resin composition containing an acrylic-based monomer; mixing a Boswellia extract in the resin composition; and injecting the mixture into a mold to form a contact lens.

In the first step, a resin composition containing an acrylic-based monomer is prepared.

Examples of the acrylic-based monomer may include, but are not limited to, acrylic acid; methacrylic acid; acrylamide; $C_1$-$C_{15}$ saturated or unsaturated alkyl acrylate or methacrylate; $C_1$-$C_{15}$ hydroxyalkyl acrylate or methacrylate with one to three substituted hydroxyl moieties; and N,N-di($C_1$-$C_{15}$ saturated or unsaturated alkyl)acrylamide, which may be used alone or in combination.

Specifically, the acrylic-based monomer may be acrylic acid (AA), methacrylic acid (MA), acrylamide, lauryl methacrylate (LMA), hydroxyethyl methacrylate (HEMA), glycerol monomethacrylate (GMMA), N,N-dimethyl acrylamide (DMA), etc.

As for the contents of the acrylic-based monomers, which are not specifically limited, hydroxyethyl methacrylate (HEMA) may be used in an amount of 90 to 98 parts by weight, and each of other monomers may be used in an amount ranging from 0.1 to 10 parts by weight.

The hydroxyethyl methacrylate (HEMA) is a hydrogel most commonly used in soft contact lenses.

A hydrogel is a network structure in which a water-soluble polymer forms three-dimensional cross-links through

physical or chemical bonding. It does not dissolve in an aqueous environment but may contain a significant amount of water. Besides, it is easy to process into various shapes and highly biocompatible due to its high water content and physicochemical similarity to the extracellular matrix.

In accordance with an embodiment of the present invention, the resin composition may use at least two functional monomers in order to obtain polymer cavities arranged to recognize the Boswellic extract.

It is using the molecularly imprinted polymer technique, of which the principal feature is the non-covalent interaction between the Boswellia extract and the polymer cavities created by the polymerization reaction. The Boswellia extract recognized by the polymer cavities may be gradually released from the contact lens.

Although the contents of the functional monomers are not specifically limited, the resin composition may contain 90 to 98 parts by weight of hydroxyethyl methacrylate (HEMA) and 1 to 3 parts by weight of methyl methacrylate (MMA).

In accordance with an embodiment of the present invention, the resin composition may further include a hydrophilic monomer, such as N-vinylpyrrolidone (NVP) or N-methylpyrrolidone (NMP), in order to increase the water content.

The hydrophilic monomer may be contained in an amount of 0.1 to 10 parts by weight, specifically 0.1 to 8 parts by weight, more specifically 0.1 to 5 parts by weight, with respect to 100 parts by weight of the resin composition.

The content of the hydrophilic monomer less than 0.1 part by weight may cause a deterioration of the wettability, whereas the content of the hydrophilic monomer greater than 10 parts by weight may lower the tensile strength.

In accordance with an embodiment of the present invention, the resin composition may further include a silicone compound, in which case, silicone hydrogel lenses can be fabricated.

In the present invention, the silicone compound is a hydrocarbon compound containing a silicon (Si) atom. To the silicon (Si) atom may be attached a substituent, such as a hydroxyl group, a C1-C10 alkoxy group, an amide group, an ester group, or a siloxy group.

Examples of the silicone compound may include, but are not limited to, 2-(trimethylsilyloxy)ethyl methacrylate, tris(3-methacryloxypropyl)silane, 3-tris(trimethylsiloxy)silylpropyl methacrylate, or 4-methacryloxybutyl terminated polydimethyl siloxane.

The silicone compound may be contained in an amount of 0.1 to 50 parts by weight with respect to 100 parts by weight of the resin composition. The content of the silicon compound greater than 50 parts by weight may result in deterioration of tensile strength and wettability.

In accordance with an embodiment of the present invention, the resin composition may further include a cross-linking agent or an initiator.

In accordance with an embodiment of the present invention, examples of the cross-linking agent may include, but are not limited to, ethylene glycol dimethacrylate (EGDMA), diethylene glycol methacrylate (DEGMA), divinylbenzene, and trimethylolpropane trimethacrylate (TMPTMA).

The cross-linking agent may be contained in an amount of 0.1 to 5 parts by weight with respect to 100 parts by weight of the resin composition. Specifically, the content of the cross-linking agent may be 0.1 to 3 parts by weight, more specifically 0.1 to 1 parts by weight. The content of the cross-linking agent greater than 1 part by weight may not only result in a failure to create polymer cavities necessary to the molecularly imprinted polymer technique but also adversely affect the optical field of vision, flexibility and water content of the contact lens.

In accordance with an embodiment of the present invention, examples of the initiator may include, but are not limited to, azodiisobutyronitrile (AIBN), benzoin methyl ether (BME), 2,5-dimethyl-2,5-di-(2-ethylhexanoylperoxy)hexane, and dimethoxyphenylacetophenone (DMPA).

The initiator may be contained in an amount of 0.001 to 5 parts by weight with respect to 100 parts by weight of the resin composition. Specifically, the content of the initiator may be 0.01 to 1 part by weight, more specifically 0.01 to 0.5 part by weight. The content of the initiator greater than 5 parts by weight may not only result in a failure to create polymer cavities necessary to the molecularly imprinted polymer technique but also adversely affect the optical field of vision, flexibility and water content of the contact lens.

In the second step, a Boswellia extract is mixed in the resin composition to prepare a mixture.

The specific features and the manufacturing method of the Boswellia extract are as described above. In accordance with an embodiment of the present invention, as stated above, a boswellic acid may be used as the Boswellia extract.

The Boswellia extract may be contained in an amount of 0.001 to 1 part by weight with respect to 100 parts by weight of the resin composition. The content of the Boswellia extract less than 0.001 part by weight may cause a risk of deteriorating the contact fit and antibacterial performance of the contact lens, whereas the content of the Boswellia extract greater than 1 part by weight may reduce the tensile strength and oxygen permeability of the contact lens.

In the third step, the mixture is injected into a mold to form a contact lens.

In accordance with an embodiment of the present invention, this procedure may include, but is not limited to, using a technique of spin casting or cast molding.

Spin casting is the process that involves introducing the resin composition used as a casting material for lens

into a spinning mold, making the resin composition pulled by the centrifugal force and spread on the surface of the mold, and activating polymerization under UV radiations or heat. The shape of the lens may be determined according to the shape of the mold, the spinning rate, and the physical properties and injected amount of the resin composition.

Cast molding is the process by which the resin composition used as a casting material for lens is poured into a mold having the shapes of the front and back curves of the lens and then subjected to polymerization by UV radiations or heat.

In an embodiment of the present invention, the molding process may be performed at 60 to 120 °C for 20 minutes to 5 hours, specifically at 80 to 100 °C for 40 minutes to 1 hour or at 100 to 120 °C for 20 to 30 minutes.

A method for manufacturing a contact lens according to another embodiment of the present invention may include: preparing a resin composition containing an acrylic-based monomer; injecting the resin composition into a mold to pre-mold a contact lens; and applying a Boswellia extract to the pre-molded contact lens by coating.

In the first step, a resin composition containing an acrylic-based monomer is prepared.

The components and contents of the resin composition containing an acrylic-based monomer are as described above.

In the second step, the resin composition is injected into a mold to pre-mold a contact lens.

As stated above, the molding process may use spin casting or cast molding.

In the third step, a Boswellia extract is applied to the pre-molded contact lens by coating.

The step of applying a Boswellia extract by coating may include preparing a coating solution with the Boswellia extract and applying the Boswellia coating solution.

In accordance with an embodiment of the present invention, the Boswellia coating solution may contain 80 to 90 parts by weight of purified water, 0.1 to 2 parts by weight of a hydrophilic polymer, 1 to 5 parts by weight of polyethylene glycol, 0.01 to 5 parts by weight of a Boswellia extract, and 1 to 5 parts by weight of a pH adjusting agent. The Boswellia coating solution may be prepared by mixing all the ingredients together and applying agitation to the mixture. The agitation may involve, but is not limited to, agitating at a rate of 100 to 500 rpm for 10 to 60 minutes.

In accordance with an embodiment of the present invention, the Boswellia extract may be a boswellic acid, which may include at least one of the compounds represented by the afore-mentioned chemical formulas 1 to 8.

In accordance with an embodiment of the present invention, examples of the hydrophilic polymer may include, but are not limited to, polyvinyl alcohol, polyvinyl pyrrolidone, sodium hyaluronate, choline phosphate polymer, or cellulose acetate.

In accordance with an embodiment of the present invention, the coating may be performed using a method known in the art, examples of which method may include, but are not limited to, dip coating.

The contact lens thus manufactured may be filled with saline water through hydration and inspection processes and then finished into a complete product through sterilizing and packaging processes.

The contact lens according to an embodiment of the present invention contains a Boswellia extract to secure a good contact fit and high stability.

The contact lens manufactured according to an embodiment of the present invention also contains a Boswellia extract, thereby preventing adherence of bacteria when worn on the user's eye. The Boswellia extract is gradually released from the contact lens and acts as an antibacterial agent to prevent bacterial infection.

The contact lens according to an embodiment of the present invention is coated with the Boswellia extract, which may improve the slip performance of the contact's surface. This makes it possible to reduce a risk of damaging the eyelids and the cornea, provide a protective coating for the surfaces of the eyelids and the cornea to increase the user's eye comfort, and form a stabilized tear film.

Besides, it reduces the protein precipitates adhered to the contact lens to eventually prevent deterioration of the contact's water content and oxygen permeability and results in a decrease in the contact angle, preventing contact lens wear uncomfortable.

Hereinafter, the present invention will be described in further detail with reference to the examples according to an embodiment of the present invention, which examples are not intended to limit the scope of the present invention.

**[Examples]**

Example 1

94 parts by weight of 2-hydroxyethyl methacrylate, 0.1 part by weight of methyl methacrylate (MMA), and 5 parts by weight of N-vinyl pyrrolidone (NVP) were mixed together, and 0.01 part by weight of a Boswellia extract. Then, 0.1 part by weight of ethylene glycol dimethacrylate (EGDMA) as a cross-linking agent and 0.01 part by weight of izobisisobutyronitrile (AIBN) as an initiator were mixed in at 40 °C.

The mixture was introduced into a mold and subjected to a polymerization reaction in an oven at 100 °C for 30 minutes and then a heat treatment. The contact lens sample thus obtained was hydrated with distilled water and stored

in saline water.

Example 2

The procedures were performed in the same manner as described in Example 1, excepting that a boswellic acid (β-boswellic acid) was used in place of the Boswellia extract to fabricate a contact lens sample.

Examples 3 and 4

The procedures were performed in the same manner as described in Examples 1 and 2, excepting that the resin composition containing 90 parts by weight of 2-hydroxyethyl methacrylate, 1 part by weight of N,N-dimethylacrylamide and 20 parts by weight of 4-methacryloxybutyl terminated polydimethylsiloxane was used to fabricate a contact lens sample.

Example 5

The procedures were performed in the same manner as described in Example 1, excepting that the mixture was prepared without using a Boswellia extract. The mixture was introduced into a mold for pre-molding a contact lens, and a Boswellia coating solution was applied. The Boswellia coating solution was heated up to 90 °C and applied onto the pre-molded contact lens through dip coating for 20 minutes. Following the dip coating, the contact lens was subjected to a heat treatment, hydrated with distilled water and stored in saline water.

Comparative Example

The procedures were performed in the same manner as described in Example 1, excepting that the mixture was prepared without using a Boswellia extract. The mixture was introduced into a mold and subjected to a polymerization reaction in an oven at 100 °C for 30 minutes and then a treat treatment. The contact lens thus obtained was hydrated with distilled water and stored in saline water.

[Evaluations]

1) Visible light transmittance

The visible light transmittance was measured according to the ISO 18369-3 standards. All the contact lenses according to the examples exhibited transparency of 95% or above at wavelengths of visible light.

2) Water content

The water content was measured according to the ISO 18369-4 standards. The following equation was given to calculate the water content. The measurement of water content was carried out three times per sample, and the average value of the measurements was used. All the contact lenses according to the examples exhibited a water content of 35 to 65 %.

$$\text{Water content (\%)} = (\text{Weight of hydrated lens} - \text{Weight of dried lens}) / (\text{Weight of hydrated lens}) \times 100$$

3) Contact angle

The contact angle was measured according to the ISO 18369 standards. All the contact lenses according to the examples had a contact angle of 45° or below.

4) Tensile strength

The tensile strength was measured according to the ASTM D955 standards. All the contact lenses according to the examples had a tensile strength of 0.1 kgf or above.

5) Oxygen permeability

The contact lenses were put in a standard physiological saline solution (PBS), stored at room temperature (20°C±2°C) for 24 hours, treated at the same temperature of the eye (35°C±0.5°C) for at least 2 hours, and put into use for the tests. Among the contact lenses according to the examples, the hydrogel lens had an oxygen transmissibility of 10 to 50 Dk/t and the silicone-hydrogel lens had an oxygen transmissibility of 50 to 120 Dk/t.

6) Antibacterial capacity

The contact lenses according to the examples were measured in regards to the antibacterial capacity against *Staphylococcus aureus.* The control was the comparative example. In relation to the control, the contact lenses of the examples had the average number of colonies reduced by at least 80%, showing high antibacterial capacity.

Although the exemplary embodiments of the present invention have been described, it is understood that the present invention should not be limited to these exemplary embodiments but various changes and modifications can be made by one ordinary skilled in the art within the spirit and scope of the present invention as hereinafter claimed.

The contact lens according to an embodiment of the present invention can secure a good contact fit and high stability by containing a Boswellia extract.

The contact lens manufactured according to an embodiment of the present invention also contains a Boswellia extract, thereby preventing adherence of bacteria when worn on the user's eye.

The contact lens according to an embodiment of the present invention is coated with the Boswellia extract, which improves the slip performance of the contact's surface.

## Claims

1. A contact lens comprising a resin composition containing an acrylic-based monomer and a Boswellia extract.

2. The contact lens according to claim 1, wherein the Boswellia extract comprises at least one of boswellic acids represented by the following chemical formulas:

[Chemical Formula 1]

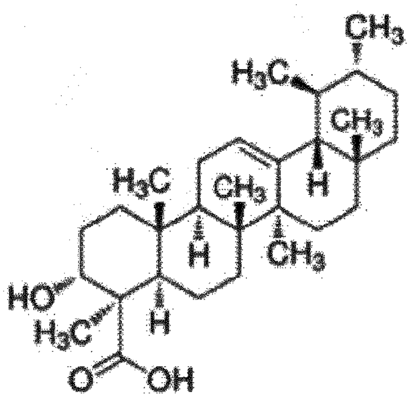

[Chemical Formula 2]

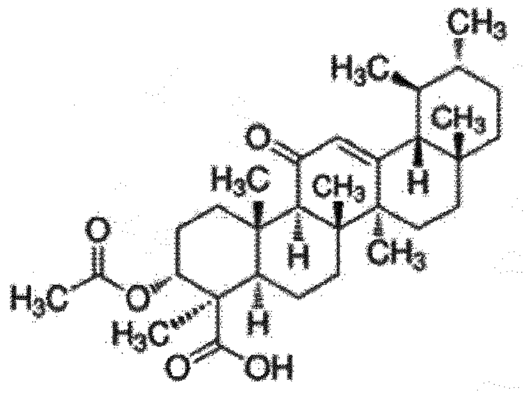

[Chemical Formula 3]

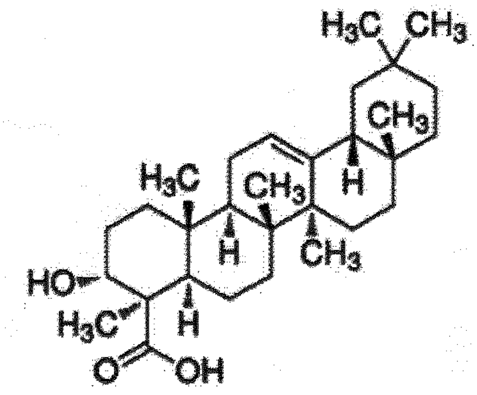

[Chemical Formula 4]

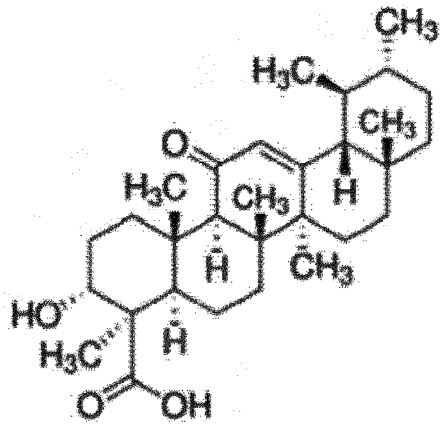

[Chemical Formula 5]

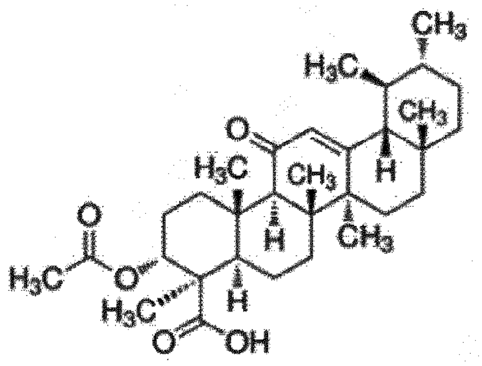

[Chemical Formula 6]

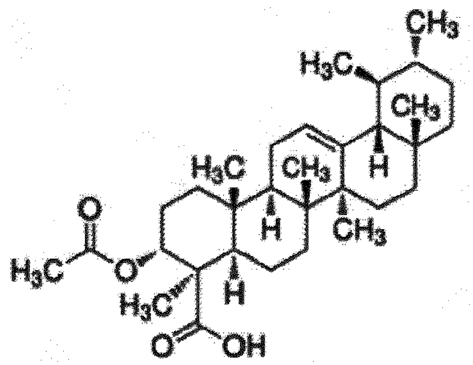

[Chemical Formula 7]

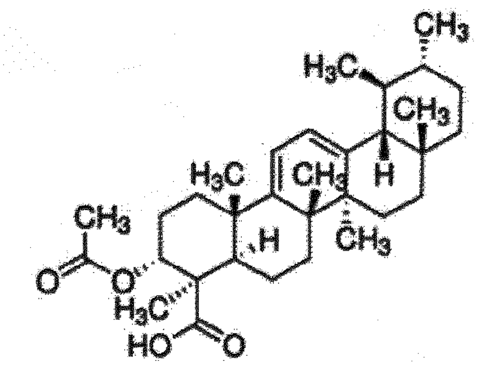

[Chemical Formula 8]

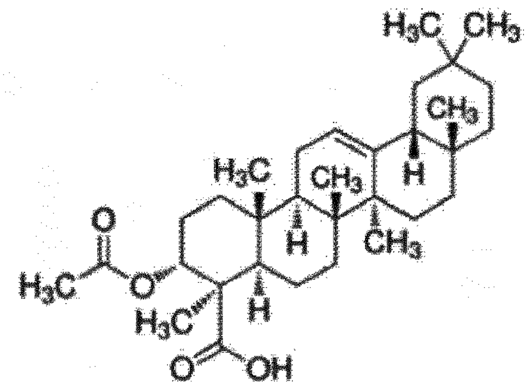

**3.** The contact lens according to claim 1, wherein the acrylic-based monomer comprises at least one selected from the group consisting of acrylic acid; methacrylic acid; acrylamide; $C_1$-$C_{15}$ saturated or unsaturated alkyl acrylate or methacrylate; $C_1$-$C_{15}$ hydroxyalkyl acrylate or methacrylate with one to three substituted hydroxyl moieties; and N,N-di($C_1$-$C_{15}$ saturated or unsaturated alkyl)acrylamide.

4. The contact lens according to claim 1, wherein the resin composition further comprises at least one hydrophilic monomer selected from N-vinylpyrrolidone (NVP) and N-methylpyrrolidone (NMP).

5. The contact lens according to claim 1, wherein the resin composition comprises a silicone compound.

6. The contact lens according to claim 1, wherein the resin composition comprises at least one silicone compound selected from the group consisting of 2-(trimethylsilyloxy)ethyl methacrylate, tris(3-methacryloxypropyl)silane, 3-tris(trimethylsiloxy)silylpropyl methacrylate, and 4-methacryloxybutyl terminated polydimethyl siloxane.

7. A method for manufacturing a contact lens, comprising:

preparing a resin composition comprising an acrylic-based monomer;
mixing a Boswellia extract in the resin composition; and
injecting the mixture into a mold to form a contact lens.

8. A method for manufacturing a contact lens, comprising:

preparing a resin composition comprising an acrylic-based monomer;
injecting the resin composition into a mold to pre-mold a contact lens; and
applying a Boswellia extract to the pre-molded contact lens by coating.

9. The method according to claim 7 or 8, wherein the Boswellia extract comprises at least one of boswellic acids represented by the following chemical formulas:

[Chemical Formula 1]

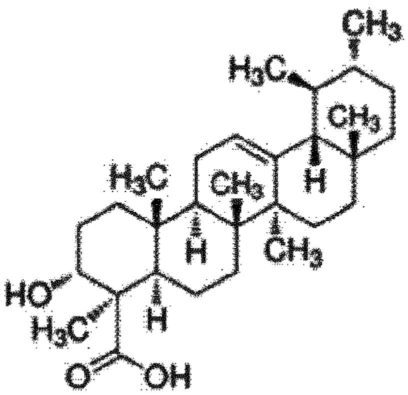

[Chemical Formula 2]

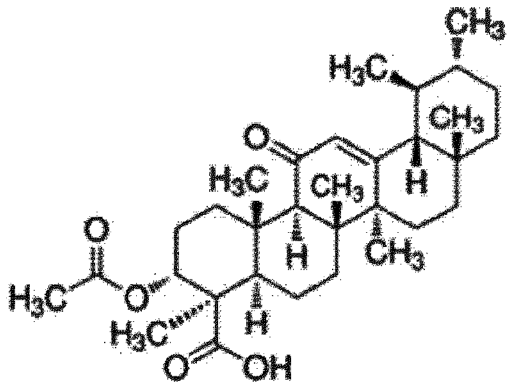

[Chemical Formula 3]

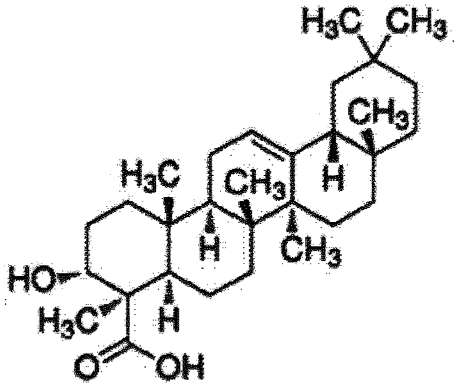

[Chemical Formula 4]

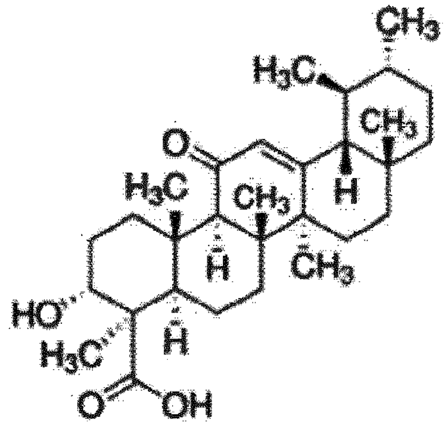

[Chemical Formula 5]

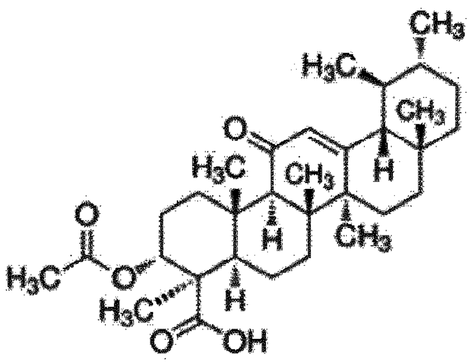

[Chemical Formula 6]

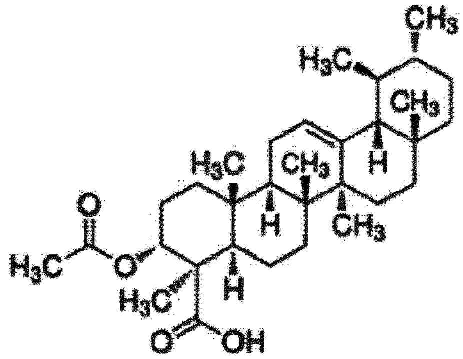

[Chemical Formula 7]

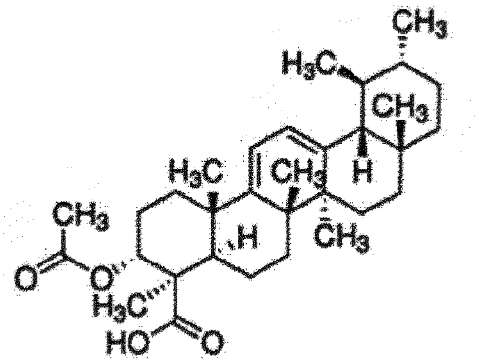

[Chemical Formula 8]

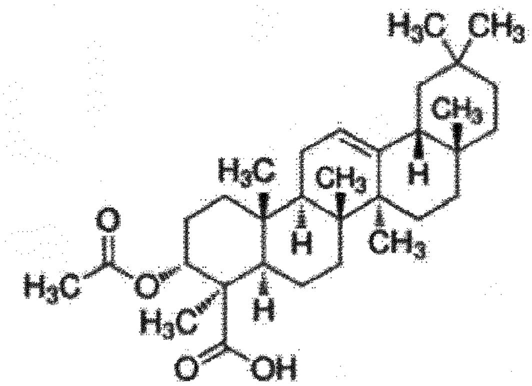

**10.** The method according to claim 8, wherein the step of applying the Boswellia extract by coating is conducted using a Boswellia coating solution comprising 80 to 90 parts by weight of purified water, 0.1 to 2 parts by weight of a hydrophilic polymer, 1 to 5 parts by weight of polyethylene glycol, 0.01 to 5 parts by weight of a Boswellia extract, and 1 to 5 parts by weight of a pH adjusting agent.

**11.** A contact lens manufactured by the method according to any one of claims 7 to 10.

## INTERNATIONAL SEARCH REPORT

International application No.
**PCT/KR2020/014709**

**A. CLASSIFICATION OF SUBJECT MATTER**

**G02C 7/04**(2006.01)i; **A61L 12/08**(2006.01)i; **C08K 5/12**(2006.01)i; **C08K 5/5415**(2006.01)i; **C08K 5/092**(2006.01)i; **C08L 33/14**(2006.01)i; **C08J 7/04**(2006.01)i; **C09D 133/14**(2006.01)i

According to International Patent Classification (IPC) or to both national classification and IPC

**B. FIELDS SEARCHED**

Minimum documentation searched (classification system followed by classification symbols)

G02C 7/04(2006.01); A01N 25/34(2006.01); A61K 31/35(2006.01); A61K 31/704(2006.01); A61K 36/324(2006.01); A61K 9/48(2006.01); A61L 12/00(2006.01); A61P 27/02(2006.01); G01N 21/64(2006.01); G01N 27/414(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 보스웰리아(boswellia), 추출물(extract), 콘택트 렌즈(contact lenses), 아크릴계 단량체(acrylic monomers), 수지(resin)

**C. DOCUMENTS CONSIDERED TO BE RELEVANT**

| Category* | Citation of document, with indication, where appropriate, of the relevant passages | Relevant to claim No. |
|---|---|---|
| A | KR 10-1953474 B1 (FOUNDATION FOR RESEARCH AND BUSINESS, SEOUL NATIONAL UNIVERSITY OF SCIENCE AND TECHNOLOGY) 28 February 2019 (2019-02-28)<br>See abstract; and paragraphs [0060], [0061] and [0064]. | 1-10 |
| A | KR 10-2014-0022108 A (BVW HOLDING AG) 21 February 2014 (2014-02-21)<br>See abstract; claim 49; and paragraphs [0056], [0123], [0168], [0169] and [0222]-[0232]. | 1-10 |
| A | KR 10-2009-0115165 A (NOVARTIS AG) 04 November 2009 (2009-11-04)<br>See claims 1-21. | 1-10 |
| A | US 2013-0338039 A1 (MAZED, M. A. et al.) 19 December 2013 (2013-12-19)<br>See claims 1-20; and tables 7A and 7B. | 1-10 |
| A | KR 10-2009-0005797 A (NEW BIO CO., LTD. et al.) 14 January 2009 (2009-01-14)<br>See entire document. | 1-10 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 March 2021** | **30 March 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.
**PCT/KR2020/014709**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☑ Claims Nos.: **11**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2020/014709**

| Patent document cited in search report | Publication date (day/month/year) | Patent family member(s) | Publication date (day/month/year) |
|---|---|---|---|
| KR 10-1953474 B1 | 28 February 2019 | None | |
| KR 10-2014-0022108 A | 21 February 2014 | CN 103889434 A | 25 June 2014 |
| | | CN 103889434 B | 15 February 2017 |
| | | EP 2723357 A1 | 30 April 2014 |
| | | EP 3597206 A1 | 22 January 2020 |
| | | HK 1198746 A1 | 05 June 2015 |
| | | JP 2014-517070 A | 17 July 2014 |
| | | JP 2017-222660 A | 21 December 2017 |
| | | JP 6302835 B2 | 28 March 2018 |
| | | TW 201306827 A | 16 February 2013 |
| | | TW I465243 B | 21 December 2014 |
| | | US 2014-0301971 A1 | 09 October 2014 |
| | | WO 2012-177825 A1 | 27 December 2012 |
| KR 10-2009-0115165 A | 04 November 2009 | CA 2678598 A1 | 30 April 2009 |
| | | CA 2678598 C | 03 February 2015 |
| | | EP 2115499 A2 | 11 November 2009 |
| | | EP 2115499 B1 | 11 November 2015 |
| | | HU E027476 T2 | 28 September 2016 |
| | | JP 2011-513767 A | 28 April 2011 |
| | | JP 5675111 B2 | 25 February 2015 |
| | | US 2008-203592 A1 | 28 August 2008 |
| | | WO 2009-055082 A2 | 30 April 2009 |
| | | WO 2009-055082 A3 | 30 July 2009 |
| US 2013-0338039 A1 | 19 December 2013 | US 10154326 B2 | 11 December 2018 |
| | | US 10382848 B2 | 13 August 2019 |
| | | US 10529003 B2 | 07 January 2020 |
| | | US 10540704 B2 | 21 January 2020 |
| | | US 10595104 B2 | 17 March 2020 |
| | | US 10638208 B2 | 28 April 2020 |
| | | US 2009-0252758 A1 | 08 October 2009 |
| | | US 2009-0252796 A1 | 08 October 2009 |
| | | US 2010-0021533 A1 | 28 January 2010 |
| | | US 2010-0073202 A1 | 25 March 2010 |
| | | US 2011-0158653 A1 | 30 June 2011 |
| | | US 2011-0274680 A1 | 10 November 2011 |
| | | US 2011-0293278 A1 | 01 December 2011 |
| | | US 2012-0265596 A1 | 18 October 2012 |
| | | US 2015-0382089 A1 | 31 December 2015 |
| | | US 2016-0004298 A1 | 07 January 2016 |
| | | US 2016-0212512 A9 | 21 July 2016 |
| | | US 2016-0334866 A9 | 17 November 2016 |
| | | US 2017-0006363 A1 | 05 January 2017 |
| | | US 2017-0018688 A1 | 19 January 2017 |
| | | US 2017-0221032 A1 | 03 August 2017 |
| | | US 2017-0272847 A1 | 21 September 2017 |
| | | US 2017-0316487 A1 | 02 November 2017 |
| | | US 2019-0124426 A1 | 25 April 2019 |
| | | US 2019-0373347 A1 | 05 December 2019 |
| | | US 2019-0394545 A1 | 26 December 2019 |
| | | US 8017147 B2 | 13 September 2011 |

Form PCT/ISA/210 (patent family annex) (July 2019)

**INTERNATIONAL SEARCH REPORT**
**Information on patent family members**

International application No.
**PCT/KR2020/014709**

| Patent document cited in search report | Publication date (day/month/year) | Patent family member(s) | Publication date (day/month/year) |
|---|---|---|---|
| | | US 8073331 B1 | 06 December 2011 |
| | | US 8548334 B2 | 01 October 2013 |
| | | US 9426545 B2 | 23 August 2016 |
| | | US 9557271 B2 | 31 January 2017 |
| | | US 9697556 B2 | 04 July 2017 |
| | | US 9723388 B2 | 01 August 2017 |
| | | US 9823737 B2 | 21 November 2017 |
| | | US 9923124 B2 | 20 March 2018 |
| KR 10-2009-0005797 A | 14 January 2009 | KR 10-0878053 B1 | 15 January 2009 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 100878053
- KR 101953474


### Non-patent literature cited in the description


- *Journal of the Korean Society of Food Science and Nutrition,* vol. 43 (5), 20145, , 631-640
- **FRANCESCA BORRELIN.** *British Journal of Pharmacy*